# NEUE EUROPÄISCHE PATENTSCHRIFT

(11) **EP 0 903 348 B2**
(45) Veröffentlichungstag und Bekanntmachung des Hinweises auf die Entscheidung über den Einspruch: **27.08.2008**
(45) Hinweis auf die Patenterteilung: 05.06.2002
(21) Anmeldenummer: 98121523.9
(22) Anmeldetag: 18.11.1996
(51) Int. Cl.: C07D 277/30, C07D 417/06, C07D 493/04, A61K 31/425, A01N 43/78, C07D 313/00, C07D 303/00

(54) **Epothilon-Derivate und deren Herstellung**
Epothilone derivatives and preparation thereof
Dérivés d'epothilone et leur préparation

(30) Priorität: 17.11.1995 DE 19542986; 25.09.1996 DE 19639456
(43) Veröffentlichungstag der Anmeldung: 24.03.1999
(62) Teilanmeldung aus: 96939097.0
(73) Patentinhaber: Gesellschaft für Biotechnologische Forschung mbH (GBF), D-38124 Braunschweig (DE)
(72) Erfinder: Höfle, Gerhard, Prof. Dr., 38124 Braunschweig (DE); Kiffe, Michael, Dr., 38124 Braunschweig (DE)
(74) Vertreter: Boeters, Hans Dietrich

(56) Entgegenhaltungen:
- WO-A-93/10121
- K.C.NICOLAOU et al. Angew.Chem.Int.Ed. 37 (1998) 2015-2045

## Beschreibung

Die vorliegende Erfindung betrifft ein Verfahren zur Herstellung eines Epothilonderivats der nachfolgend dargestellten allgemeinen Formel 7

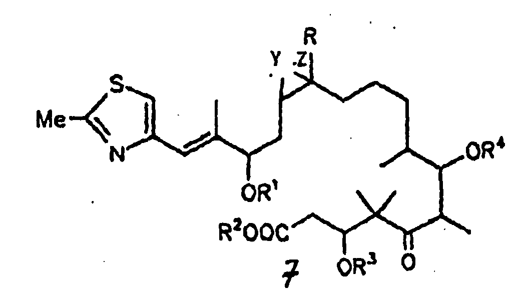

In der vorstehenden Formel 7 bedeuten:
- R =: H oder C₁-Alkyl;
- R¹, R², R³, R⁴ =: H, C₁₋₆-Alkyl,
C₁₋₆-Acyl
Benzoyl,
C₁₋₄-Trialkylsilyl,
Benzyl, oder
Phenyl, wobei Benzyl und Phenyl
C₁₋₆-Alkoxy-,
C₆-Alkyl, Hydroxy- und Halogen-substituiertes Benzyl bzw. Phenyl sein können;
wobei es sich bei den in den Resten enthaltenen Alkyl- bzw. Acylgruppen um geradkettige oder verzweigte Reste handelt; und
Y und Z entweder gemeinsam das O-Atom eines Epoxides oder eine der C-C-Bindungen einer C=C-Doppelbindung bilden, wobei man Epothilon vom Typ A, B, C oder D, oder deren 3-OH- oder 7-OH-geschützte Derivate, bei denen in Form ihres Lactons gemäß der vorstehenden allgemeinen Formel 7
R = H oder C₁₋₄-Alkyl und R³ und R⁴ = H, C₁₋₆-Alkyl, C₁₋₆-Acyl, Benzoyl, C₁₋₄-Trialkylsilyl, Benzyl oder Phenyl, wobei Benzyl und Phenyl C₁₋₆-Alkoxy-, C₆-Alkyl-, Hydroxy- und halogensubstituiert sein können; und wobei es sich bei den in den Resten enthaltenen Alkyl- bzw. Acylgruppen um geradkettige oder verzweigte Reste handelt; und Y und Z entweder gemeinsam das O-Atom eines Epoxids oder eine der C-C-Bindungen einer C=C-Doppelbindung bilden,
einer basischen Hydrolyse, z.B. mit NaOH in MeOH oder MeOH/Wasser, oder einer enzymatischen Hydrolyse unterwirft, insbesondere mit Esterasen oder Lipasen.

Die vorliegende Erfindung betrifft ferner um Epothilonderivate der nachfolgend dargestellten allgemeinen Formel 7.

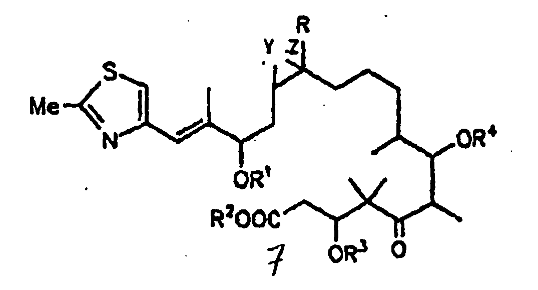

In der vorstehenden Formel 7 bedeuten:
- R =: H oder C₁-Alkyl;
- R¹, R², R³, R⁴ =: H, C₁₋₆-Alkyl,
C₁₋₆-Acyl
Benzoyl,
C₁₋₄-Trialkylsilyl,
Benzyl, oder
Phenyl, wobei Benzyl und Phenyl
C₁₋₆-Alkoxy-,
C₆-Alkyl, Hydroxy- und Halogen-substituiertes Benzyl bzw. Phenyl sein können;
wobei es sich bei den in den Resten enthaltenen Alkyl- bzw. Acylgruppen um geradkettige oder verzweigte Reste handelt; und
Y und Z entweder gemeinsam das O-Atom eines Epoxides oder eine der C-C-Bindungen einer C=C-Doppelbindung bilden.

Die vorliegende Erfindung betrifft ferner ein Verfahren zur Herstellung einer Epothilonderivate der Formel 1

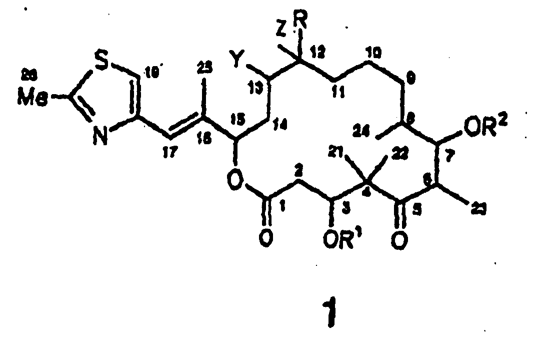

In der vorstehenden Formel 1 bedeuten:
- R =: H oder C₁-Alkyl;
- R¹, R² =: H, C₁₋₆-Alkyl,
C₁₋₆-Acyl
Benzoyl,
C₁₋₄-Trialkylsilyl,
Benzyl, oder
Phenyl, wobei Benzyl und Phenyl
C₁₋₆-Alkoxy-,
C₆-Alkyl-1, Hydroxy- und Halogen-substituiertes Benzyl bzw. Phenyl sein können;
wobei es sich bei den in den Resten enthaltenen Alkyl- bzw. Acylgruppen um geradkettige oder verzweigte Reste handelt; und
Y und Z entweder gemeinsam das O-Atom eines Epoxides oder eine der C-C-Bindungen einer C=C-Doppelbindung bilden, wobei man eine Verbindung der Formel 7 durch Lactonisierung nach den Mathoden von Yamaguchi (Tri- chlorbenzoylchlorid/DMAP), Corey (Aldrithiol/Triphenylphosphin) oder Kellogg (omega-Bromsäure/Caesiumcarbonat) in eine Verbindung der **Formel 1** umgewandelt. Einschlägige Arbeitsmethoden finden sich bei

Inanaga et al. in Bull. Chem. Soc. Japan, 52 (1979) 1989; Corey & Nicolaou in J. Am. Chem. Soc., 96 (1974) 5614; und Kruizinga & Kellogg in J. Am. Chem. Soc., 103 (1981) 5183.

Zur Herstellung der erfindungsgemäßen Verbindungen kann man also auch von Epothilon C oder D ausgehen, wobei zur Derivatisierung auf die nachstehend beschriebenen Derivatisierungsmethoden verwiesen werden kann. Dabei kann man die 12,13-Doppelbindung selektiv epoxidieren, beispielsweise mit Dimethyldioxiran oder einer Persäure.

Für Epothilon A und B sei verwiesen auf DE-A-41 38 042.

Die Erfindung wird im folgenden durch die Beschreibung von einigen ausgewählten Ausführungsbeispielen näher erläutert und beschrieben.

### Beispiele

### Beispiel 1:

### Verbindung 7a

20 mg (0.041 mmol) Epothilon werden in 0.5 ml Methanol gelöst, mit 0.5 ml 1 N Natronlauge versetzt und 5 Minuten bei Raumtemperatur gerührt.
Zur Aufarbeitung wird das Reaktionsgemisch mit 1 M Phosphatpuffer pH 7 versetzt und die wäßrige Phase viermal mit Ethylacetat extrahiert. Die vereinigten organischen Phasen werden mit gesättigter Natriumchlorid-Lösung gewaschen, über Natriumsulfat getrocknet und vom Lösungsmittel befreit. Die Reinigung des Rohproduktes erfolgt mit Hilfe der präparativen Schichtchromatographie (Laufmittel: Dichlormethan/Methanol, 85 : 15).
- Ausbeute:: 11 mg (52 %)
- R_{f} (Dichlormethan/Methanol, 85 : 15):: 0.92
- IR (Film):: ny = 3438 (s, br, Sch), 2971 (vs, br, Sch), 1703 (vs), 1507 (m), 1460 (s, Sch), 1383 (m, Sch), 1254 (w), 1190 (w, br, Sch), 1011 (w, br, Sch), 866 (w, br), 729 (s) cm⁻¹
- MS (20/70 eV):: m/e (%) = 423 (0.1 [M⁺]), 323 (4), 168 (89), 140 (100), 85 (31), 57 (67).
- Hochauflösung:: C₂₃H₃₇O₄NS ber.: 423.2443 für [M⁺]
gef.: 423.2410

### Beispiel 2:

### Verbindung 7b

5 mg (0.009 mmol) 7-O-Acetyl-epothilon werden in 1 ml Methanol gelöst, mit 200 µl einer ammoniakalischen Methanollösung (2 mmol NH₃/ml Methanol) versetzt und zwei Tage bei 50 °C gerührt. Zur Aufarbeitung wird das Lösungsmittel im Vakuum entfernt. Die Reinigung des Rohproduktes erfolgt mit Hilfe der präparativen Schichtchromatographie (Laufmittel: Toluol/Methanol, 90 : 10).
- Ausbeute:: 3 mg (59 %)
- R_{f} (Dichlormethan/Methanol, 90 : 10):: 0.63
- IR (Film):: ny = 3441 (m, b, Sch), 2946 (s, Sch), 1732 (vs), 1600 (w), 1451 (m), 1375 (m), 1246 (s, b, Sch), 1013 (m, b, Sch) cm⁻¹
- UV (Methanol):: lambdaₘₐₓ (lg epsilon) 211 (3.75), 247 (3.59) nm.
- MS (20/70 eV) :: m/e (%) = 567 (1 [M⁺]), 465 (4), 422 (7), 388 (5), 194 (5), 182 (7), 168 (65), 164 (17), 140 (100), 97 (10), 71 (22), 43 (27).
- Hochauflösung:: C₂₉H₄₅O₈NS ber.: 567.2866 für [M⁺]
gef.: 567.2849

### Beispiel 3:

50 mg Epothilon A werden in 20 µl Dimethylsulfoxid angelöst und mit 30 ml Phosphatpuffer (pH 7,1, 30 mM) verdünnt. Nach Zugabe von 5 mg Schweineleberesterase (Fa. Boehringer Mannheim) wird 2 Tage bei 30 °C gerührt. Man säuert mit 2 N HCl auf pH 5 an und extrahiert die Epothilonsäure 7 mit Ethylacetat. Die organische Phase wird mit Natriumsulfat getrocknet, im Vakuum zur Trockne eingedampft. Ausbeute 48 mg (96 %).

### Beispiel 4:

48 mg Epothilonsäure 7 werden in 6 ml THF abs. gelöst und unter Rühren mit 40 µl Triethylamin und 16 µl 2,4,6-Trichlorbenzoylchlorid versetzt. Nach 15 min wird vom Niederschlag abfiltriert und innerhalb von 15 min unter schnellem Rühren in eine siedende Lösung von 20 mg 4-Dimethylaminopyridin in 200 ml Toluol abs. getropft. Nach weiteren 10 min wird im Vakuum eingedampft und der Rückstand zwischen Ethylacetat/Citratpuffer (pH 4) verteilt. Der Eindampfrückstand der organischen Phase ergibt nach präparativer HPLC Trennung 15 mg Epothilon A.

### Beispiel 5:

### Epothilone C und D als Ausgangsverbindungen

### A. Produktionsstamm und Kulturbedingungen entsprechend dem Epothilon-Basispatent.

### B. Produktion mit DSM 6773

75 l Kultur werden wie im Basispatent beschrieben angezogen und zum Animpfen eines Produktionsfermenters mit 700 l Produktionsmedium aus 0.8 % Stärke, 0.2 % Glukose, 0.2 % Soyamehl, 0.2 % Hefeextrakt, 0.1 % CaCl₂ x 2H₂O, 0.1 % MgSO₄ x 7H₂O, 8 mg/l Fe-EDTA, pH = 7.4 und optional 15 l Adsorberharz Amberlite XAD-16 verwendet. Die Fermentation dauert 7 - 10 Tage bei 30 C, Belüftung mit 2 m³ Luft/h. Durch Regulierung der Drehzahl wird der pO₂ bei 30 % gehalten.

### C. Isolierung

Das Adsorberharz wird mit einem 0.7 m², 100 mesh Prozeßfilter von der Kultur abgetrennt und durch Waschen mit 3 Bettvolumen Wasser/Methanol 2:1 von polaren Begleitstoffen befreit. Durch Elution mit 4 Bettvolumen Methanol wird ein Rohextrakt gewonnen, der i. Vak. bis zum Auftreten der Wasserphase eingedampft wird. Diese wird dreimal mit dem gleichen Volumen Ethylacetat extrahiert. Eindampfen der organischen Phase ergibt 240 g Rohextrakt, der zwischen Methanol und Heptan verteilt wird, um lipophile Begleitstoffe abzutrennen. Aus der Methanolphase werden durch Eindampfen i. Vak. 180 g Raffinat gewonnen, das in drei Portionen über Sephadex LH-20 (Säule 20 x 100 cm, 20 ml/min Methanol) fraktioniert wird. Die Epothilone sind in der mit 240 - 300 min Retentionszeit eluierten Fraktion von insgesamt 72 g enthalten. Zur Trennung der Epothilone wird in drei Portionen an Lichrosorb RP-18 (15 µm, Säule 10 x 40 cm, Laufmittel 180 ml/min Methanol/Wasser 65:35) chromatographiert. Nach Epothilon A und B werden mit Rₜ = 90-95 min Epothilon C und 100-110 min Epothilon D eluiert und nach Eindampfen i. Vak. in einer Ausbeute von jeweils 0.3 g als farblose Öle gewonnen.

### D. Physikalische Eigenschaften

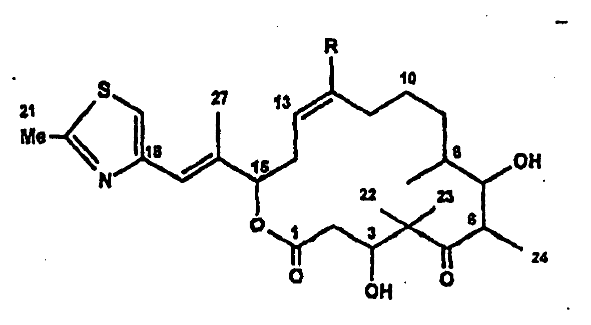

Epothilon C R = H
Epothilon D R = CH₃

### Epothilon C

C₂₆H₃₉NO₅S [477]
ESI-MS: (positiv Ionen): 478.5 für [M+H]⁺

### 1H und 13C siehe NMR-Tabelle

DC:R_{f} = 0,82
- DC-Alufolie 60 F 254 Merck, Laufmittel:: Dichlormethan/Methanol = 9:1
- Detektion:: UV-Löschung bei 254 nm. Ansprühen mit Vanillin-Schwefelsäure-Reagenz, blau-graue Anfärbung beim Erhitzen auf 120 °C.
HPLC:Rₜ = 11,5 min
Säule: Nucleosil 100 C-18 7µm, 125 x 4 mm
Laufmittel: Methanol/Wasser = 65:35
Fluß: 1ml/min
Detection: Diodenarray

### Epothilon D

C₂₇H₄₁NO₅S [491]
ESI-MS: (positiv Ionen): 492,5 für [M+H]⁺

### 1H und 13C siehe NMR-Tabelle

DC:Rf = 0,82
- DC-Alufolie 60 F 254 Merck, Laufmittel:: Dichlormethan/Methanol = 9:1
- Detektion:: UV-Löschung bei 254 nm. Ansprühen mit Vanillin-Schwefelsäure-Reagenz, blau-graue Anfärbung beim Erhitzen auf 120 °C.
HPLC:Rₜ = 15,3 min
Säule: Nucleosil 100 C-18 7µm, 125 x 4 mm
Laufmittel: Methanol/Wasser = 65:35
Fluß: 1ml/min
Detection: Diodenarray

**Tabelle:**

| ¹H-und ¹³C-NMR Daten von Epothilon C und Epothilon D in [D₆]DMSO bei 300 MHz | | | | | | |
|---|---|---|---|---|---|---|
| | Epothilon C | | | Epothilon D | | |
| H-Atom | δ (ppm) | C-Atom | δ (ppm) | δ (ppm) | C-Atom | δ (ppm) |
| | | 1 | 170.3 | | 1 | 170.1 |
| 2-Ha | 2.38 | 2 | 38.4 | 2.35 | 2 | 39.0 |
| 2-Hb | 2.50 | 3 | 71.2 | 2.38 | 3 | 70.8 |
| 3-H | 3.97 | 4 | 53.1 | 4.10 | 4 | 53.2 |
| 3-OH | 5.12 | 5 | 217.1 | 5.08 | 5 | 217.4 |
| 6-H | 3.07 | 6 | 45.4 | 3.11 | 6 | 44.4 |
| 7-H | 3.49 | 7 | 75.9 | 3.48 | 7 | 75.5 |
| 7-OH | 4.46 | 8 | 35.4 | 4.46 | 8 | 36.3 |
| 8-H | 1.34 | 9 | 27.6 | 1.29 | 9 | 29.9 |
| 9-Ha | 1.15 | 10 | 30.0 | 1.14 | 10 | 25.9 |
| 9-Hb | 1.40 | 11 | 27.6 | 1.38 | 11 | 31.8* |
| 10-Ha | 1.15* | 12 | 124.6 | 1.14* | 12 | 138.3 |
| 10-Hb | 1.35* | 13 | 133.1 | 1.35* | 13 | 120.3 |
| 11-Ha | 1.90 | 14 | 31.1 | 1.75 | 14 | 31.6* |
| 11-Hb | 2.18 | 15 | 76.3 | 2.10 | 15 | 76.6 |
| 12-H | 5.38** | 16 | 137.3 | | 16 | 137.2 |
| 13-H | 5.44** | 17 | 119.1 | 5.08 | 17 | 119.2 |
| 14-Ha | 2.35 | 18 | 152.1 | 2.30 | 18 | 152.1 |
| 14-Hb | 2.70 | 19 | 117.7 | 2.65 | 19 | 117.7 |
| 15-H | 5.27 | 20 | 164.2 | 5.29 | 20 | 164.3 |
| 17-H | 6.50 | 21 | 18.8 | 6.51 | 21 | 18.9 |
| 19-H | 7.35 | 22 | 20.8 | 7.35 | 22 | 19.7 |
| 21-H₃ | 2.65 | 23 | 22.6 | 2.65 | 23 | 22.5 |
| 22-H₃ | 0.94 | 24 | 16.7 | 0.90 | 24 | 16.4 |
| 23-H₃ | 1.21 | 25 | 18.4 | 1.19 | 25 | 18.4 |
| 24-H₃ | 1.06 | 27 | 14.2 | 1.07 | 26 | 22.9 |
| 25-H₃ | 0.90 | | | 0.91 | 27 | 14.1 |
| 26-H₃ | | | | 1.63 | | |
| 27-H₃ | 2.10 | | | 2.11 | | |

| | | | | | | |
|---|---|---|---|---|---|---|
| *, ** Zuordnung vertauschbar | | | | | | |

## Patentansprüche

1. Verfahren zur Herstellung eines Epothilonderivats der Formel 7 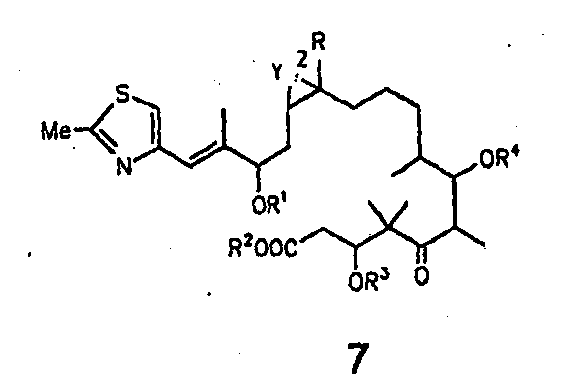 wobei R = H oder C₁-Alkyl und R¹, R², R³ und R⁴ = H, C₁₋₆-Alkyl, C₁₋₆-Acyl, Benzoyl, C₁₋₄-Trialkylsilyl, Benzyl oder Phenyl, wobei Benzyl und Phenyl C₁₋₆-Alkoxy, C₆-Alkyl-, Hydroxy- und halogensubstituiert sein können; und wobei es sich bei den in den Resten enthaltenen Alkyl- bzw. Acylgruppen um geradkettige oder verzweigte Reste handelt; und Y und Z entweder gemeinsam das O-Atom eines Epoxids oder eine der C-C-Bindungen einer C=C-Doppelbindung bilden,
**dadurch *gekennzeichnet*, daß** man Epothilon vom Typ A, B, C oder D, oder deren 3-OH- oder 7-OH-geschützte Derivate, bei denen in Form ihres Lactons gemäß der vorstehenden allgemeinen **Formel 7** R = H oder C₁-Alkyl und R³ und R⁴ = H, C₁₋₆-Alkyl, C₁₋₆-Acyl, Benzoyl, C₁₋₄-Trialkylsilyl, Benzyl oder Phenyl, wobei Benzyl und Phenyl C₁₋₆-Alkoxy-, C₆-Alkyl-, Hydroxy- und halogensubstituiert sein können; und wobei es sich bei den in den Resten enthaltenen Alkyl- bzw. Acylgruppen um geradkettige oder verzweigte Reste handelt; und Y und Z entweder gemeinsam das O-Atom eines Epoxids oder eine der C-C-Bindungen einer C=C-Doppelbindung bilden,
(a) enzymatisch hydrolysiert oder
(b) in alkalischem Medium hydrolysiert
und das Epothilonderivat der Formel 7 gewinnt und isoliert.

2. Verfahren nach Anspruch 1, **dadurch gekennzeichnet, daß** man mit einer Esterase oder Lipase hydrolysiert und das Epothilonderivat der Formel 7 gewinnt und isoliert.

3. Verfahren nach Anspruch 1, **dadurch gekennzeichnet, daß** man mit einer Esterase oder Lipase hydrolysiert und das Epothilonderivat der Formel 7 gewinnt und isoliert.

4. Epothilonderivat der Formel 7 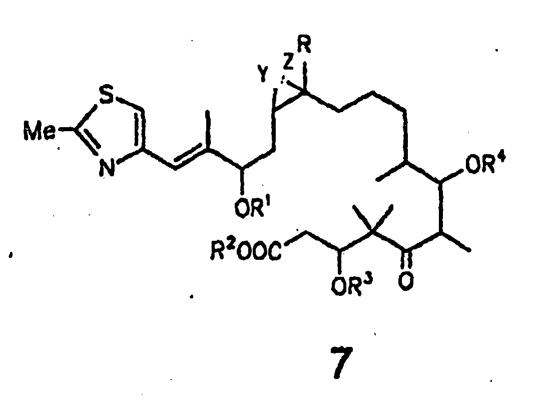 wobei R = H oder C₁-Alkyl und R¹, R², R³ und R⁴ = H, C₁₋₆-Alkyl, C₁₋₆-Acyl, Benzoyl, C₁₋₄-Trialkylsilyl, Benzyl oder Phenyl, wobei Benzyl und Phenyl C₁₋₆-Alkoxy, C₆-Alkyl-, Hydroxy- und halogensubstituiert sein können; und wobei es sich bei den in den Resten enthaltenen Alkyl- bzw. Acylgruppen um geradkettige oder verzweigte Reste handelt; und Y und Z entweder gemeinsam das O-Atom eines Epoxids oder eine der C-C-Bindungen einer C=C-Doppelbindung bilden.

5. Verfahren zur Herstellung eines Epothilonderivats der Formel 1 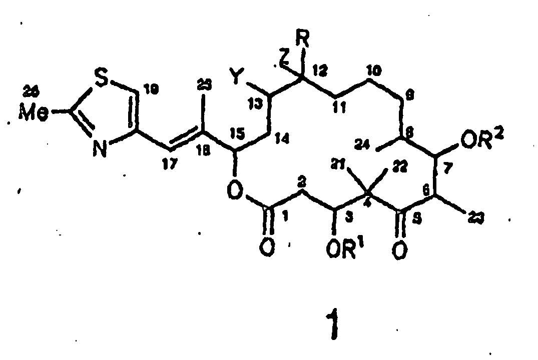 wobei R = H oder C₁-Alkyl; R¹ und R² = H, C₁₋₆-Alkyl, C₁₋₆-Acyl, Benzoyl, C₁₋₄-Trialkylsilyl, Benzyl oder Phenyl, wobei Benzyl und Phenyl C₁₋₆-Alkoxy-, C₆-Alkyl-, Hydroxy- und halogensubstituiert sein können; und wobei es sich bei den in den Resten enthaltenen Alkyl- bzw. Acylgruppen um geradkettige oder verzweigte Reste handelt; und Y und Z entweder gemeinsam das O-Atom eines Epoxids oder eine der C-C-Bindungen einer C=C-Doppelbindung bilden, **dadurch *gekennzeichnet,* daß** man ein Epothilonderivat der Formel 7 gemäß Anspruch 4 oder als Produkt des Verfahrens gemäß einem der Ansprüche 1 bis 3
(a) nach der Yamaguchi-Methode oder
(b) nach der Corey-Methode oder
(c) nach der Kellogg-Methode
lactonisiert und dadurch in das Epothilonderivat der Formel 1 umwandelt und dieses Umwandlungsprodukt isoliert.

## Claims

1. Process for the production of an epothilone derivative of formula 7 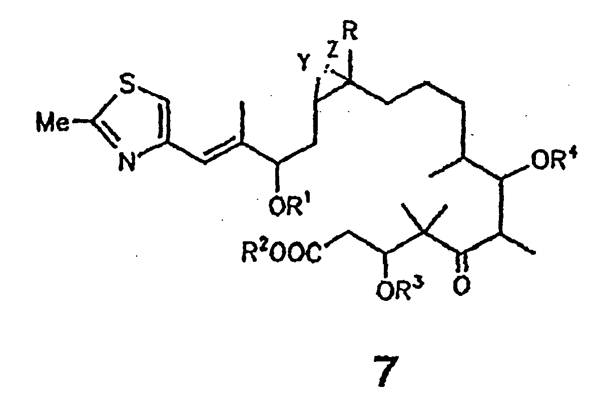 wherein
R = H or C₁-alkyl and R¹, R², R³ and R⁴ = H, C₁₋₆-alkyl, C₁₋₆-acyl, benzoyl, C₁₋₄-trialkylsilyl, benzyl or phenyl, wherein benzyl and phenyl can be substituted by C₁₋₆-alkoxy, C₆-alkyl, hydroxy and halogen; the alkyl and acyl groups contained in the radicals are straight-chain or branched radicals; and Y and Z together either form the O-atom of an epoxide or one of the C-C-bonds of a C=C-double bond, **characterized in that** an epothilone of type A, B, C or D or their 3-OH- or 7-OH- protected derivative, wherein for their state as lactone in accordance with the foregoing general formula 7 R = H, C₁-alkyl and R³ and R⁴ = H, C₁₋₆-alkyl, C₁₋₆-acyl, benzoyl, C₁₋₄-trialkylsilyl, benzyl or phenyl, wherein benzyl and phenyl can be substituted by C₁₋₆-alkoxy, C₆-alkyl, hydroxy and halogen; the alkyl and acyl groups contained in the radicals are straight-chain or branched radicals; and Y and Z together either form the O-atom of an epoxide or one of the C-C-bonds of a C=C-double bond,
a) is enzymatically hydrolysed or
b) is hydrolysed in an alkaline medium
and the epothilone derivative of formula 7 is obtained and isolated.

2. Process according to claim 1, **characterized in that** hydrolysation is carried out with an esterase or lipase and the epothilone derivative of formula 7 is obtained and isolated.

3. Process according to claim 1, **characterized in that** hydrolysation is carried out with sodium hydroxide in a methanol/water mixture and the epothilone derivative of formula 7 is obtained and isolated.

4. Epothilone derivative of formula 7 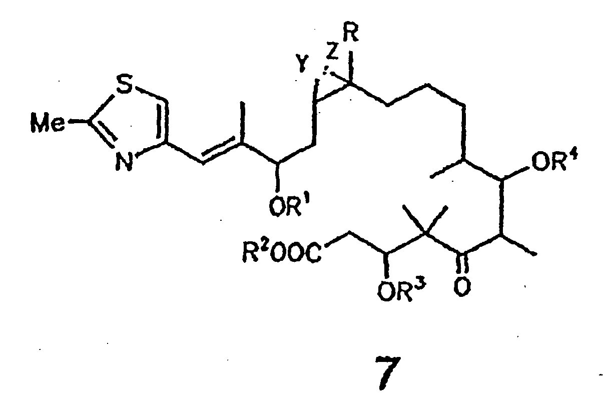 wherein
R = H or C₁-alkyl and R¹, R², R³ and R⁴ = H, C₁₋₆-alkyl, C₁₋₆-acyl, benzoyl, C₁₋₄-trialkylsilyl, benzyl or phenyl, wherein benzyl and phenyl can be substituted by C₁₋₆-alkoxy, C₆-alkyl, hydroxy and halogen; the alkyl and acyl groups contained in the radicals are straight-chain or branched radicals; and Y and Z together either form the O-atom of an epoxide or one of the C-C-bonds of a C=C-double bond.

5. Process for the preparation of an epothilon derivative of formula 1 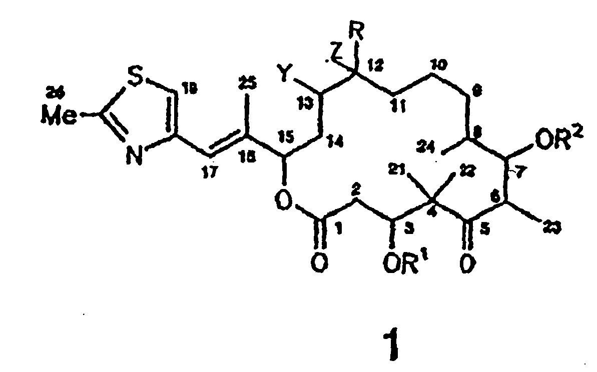 wherein
R = H, C₁-alkyl; R¹ and R² = H, C₁₋₆-alkyl, C₁₋₆-acyl, benzoyl, C₁₋₄-trialkylsilyl, benzyl or phenyl, wherein benzyl and phenyl can be substituted by C₁₋₆-alkoxy, C₆-alkyl, hydroxy and halogen; and the alkyl and acyl groups contained in the radicals are straight-chain or branched radicals; and Y and Z together either form the O-atom of an epoxide or one of the C-C-bonds of a C=C-double bond, **characterized in that** an epothilone derivative of formula 7 according to claim 4 or as a product of the process according to any of claims 1 to 3 is lactonized
(a) according to the Yamaguchi method, or
(b) according to the Corey method or
(d) according to the Kellogg method
and thereby transformed into the epothilone derivative of formula 1 and that the conversion product is isolated.

## Revendications

1. Procédé de production d'un dérivé d'épothilone de formule 7: 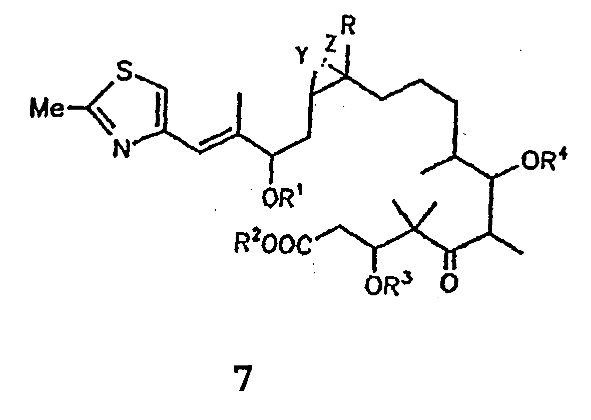 dans laquelle R représente un atome d'hydrogène ou un groupe alkyle C₁ et dans laquelle R¹, R², R³ et R⁴ représentent un atome de H, un groupe alkyle en C₁ à C₆, acyle en C₁ à C₆, benzoyle, tri (alkyle en C₁ a C₄)-silyle, benzyle ou phényle, les groupes benzyle et phényle pouvant être substitués par alcoxy en C₁ à C₆, alkyle en C₆, hydroxy et halogène; et où quant aux groupes alkyle ou acyle contenus dans les résidus, il s'agit de résidus linéaires ou ramifiés, et Y et Z forment ensemble l'atome de O d'un groupe époxyde ou bien une des liaisons C-C d'une double liaison C=C, **caractérisée en ce que** l'on hydrolyse un épothilone du type A, B, C ou D ou leurs dérivés protégés en position 3-OH or 7-OH, dans lequel sous la forme de leur lactone selon la formule générale 7 précédée R représentent un atome de H ou un groupe alkyle C₁ et dans lequels R³ et R⁴ représentent un atome de H, un groupe alkyle en C₁ à C₆, acyle en C₁ à C₆, benzoyle, tri (alkyle en C₁ à C₄)-silyle, benzyle ou phényle, les groupes benzyle et phényle pouvant être substitués par alcoxy en C₁ à C₆, alkyle en C₆, hydroxy et halogène; et où, quant aux groupes alkyle ou acyle contenus dans les résidus, il s'agit de résidus linéaires ou ramifiés; et Y et Z forment ensemble l'atome de O d'un groupe époxyde ou bien une des liaisons C-C d'une double liaison C=C,
(a) de facon enzymatique ou
(b) dans un milieu alcalin
et **en ce que** l'on obtienne et isole le dérivé épothilone de formule 7.

2. Procédé selon la revendication 1, **caractérisé en ce que** l'on hydrolyse avec une esterase ou une lipase et **en ce que** l'on obtienne et isole le dérivé épothilone de formule 7.

3. Procédé selon la revendication 1, **caractérisé en ce que** l'on hydrolyse avec de l'hydroxyde de sodium dans un mélange methanol/eau et **en ce que** l'on obtienne et isole le dérive épothilone de formule 7.

4. Dérivé épothilone de formule 7 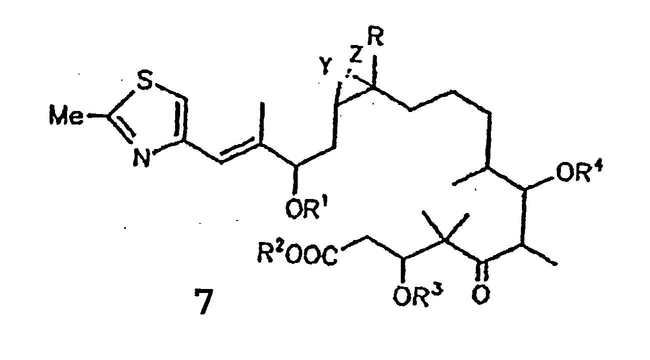 où dans laquelle R représente un atome d'hydrogène ou un groupe alkyle C₁ et dans laquelle R¹, R², R³ et R⁴ représentent un atome de H, un groupe alkyle en C₁ à C₆, acyle en C₁ à C₆, benzoyle, tri (alkyle en C₁ a C₄)-silyle, benzyle ou phényle, les groupes benzyle et phényle pouvant être substitués par alcoxy en C₁ à C₆, alkyle en C₆, hydroxy et halogène; et où quant aux groupes alkyle ou acyle contenus dans les résidus, il s'agit de résidus linéaires ou ramifiés, et Y et Z forment ensemble l'atome de O d'un groupe époxyde ou bien une des liaisons C-C d'une double liaison C=C.

5. Procédé de production d'un dérivé épothilone de formule 1 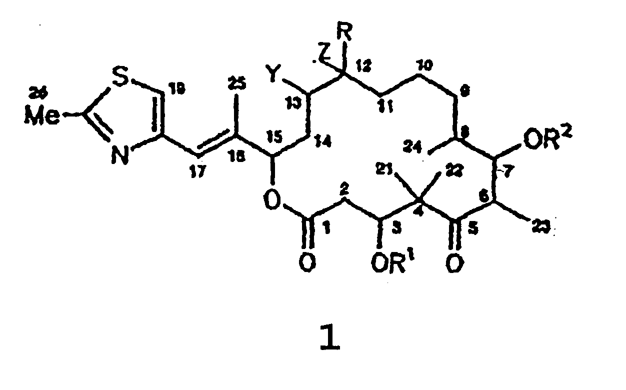 où R représente un atome d'hydrogène ou un groupe alkyle en C₁; R¹ et R² représentent un atome de H, un groupe alkyle en C₁ à C₆, acyle en C₁ à C₆, benzoyle, tri (alkyle en C₁ à C₄)-silyle, benzyle ou phényle, les groupes benzyle et phényle pouvant être substitués par alcoxy en C₁ à C₆, alkyle en C₆, hydroxy et halogène; et où quant aux groupes alkyle ou acyle contenus dans les résidus, il s'agit de résidus linéaires ou ramifiés, et Y et Z forment ensemble l'atome de O d'un groupe époxyde ou bien une des liaisons C-C d'une double liaison C=C, **caractérisé en ce que** l'on lactonise un dérivé épothilone de formule 7 selon la revendication 4 ou comme de produit du procédé selon l'une quelconque des revendications 1 à 3
(a) selon la méthode de Yamaguchi ou
(b) selon la méthode de Corey ou
(c) selon la méthode de Kellogg
et que de cette façon on le transforme en dérivé épothilone de formule 1 et qu on isole ce produit de transformation.
